(19) **Europäisches Patentamt**
**European Patent Office**
**Office européen des brevets**

(11) EP 2 623 981 B1

(12) **EUROPEAN PATENT SPECIFICATION**

(45) Date of publication and mention
of the grant of the patent:
**20.04.2016 Bulletin 2016/16**

(21) Application number: **11828425.6**

(22) Date of filing: **28.09.2011**

(51) Int Cl.:
*G01N 33/543* (2006.01)  *G01N 33/48* (2006.01)

(86) International application number:
**PCT/JP2011/005441**

(87) International publication number:
**WO 2012/042859 (05.04.2012 Gazette 2012/14)**

(54) **TESTING METHOD AND DEVICE**

TESTVERFAHREN UND -VORRICHTUNG

PROCÉDÉ ET DISPOSITIF DE TEST

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2010 JP 2010222248**

(43) Date of publication of application:
**07.08.2013 Bulletin 2013/32**

(73) Proprietor: **FUJIFILM Corporation**
**Tokyo 106-8620 (JP)**

(72) Inventors:
• **NISHIO, Tomonori**
**Ashigarakami-gun**
**Kanagawa 258-8538 (JP)**
• **UCHIDA, Mitsuaki**
**Ashigarakami-gun**
**Kanagawa 258-8538 (JP)**

(74) Representative: **Hoffmann Eitle**
**Patent- und Rechtsanwälte PartmbB**
**Arabellastraße 30**
**81925 München (DE)**

(56) References cited:
**WO-A2-03/012443    JP-A- 10 253 632
JP-A- 2010 117 290**

• **Admin: "Title: Rapid HIV Test by SD Bio-Line
Kit", , 29 January 2008 (2008-01-29),
XP055101196, Retrieved from the Internet:
URL:http://www.nacp.gov.pk/introduction/so
p/rapid-hiv-tests-by-sd-bio-line-kit.pdf [retrieved
on 2014-02-10]**
• **Gaby Vercauteren ET AL: "Multi-analyte testing
for HIV and other infections", , 25 September 2013
(2013-09-25), XP055101215, Retrieved from the
Internet:
URL:http://apps.who.int/prequal/trainingre
sources/pq_pres/meeting_WHO_UNICEF_UNFP
A_2 013/Day_2/19_Vercauteren.pdf [retrieved on
2014-02-10]**

EP 2 623 981 B1

## Description

Technical Field

The present invention relates to a test method and use of an apparatus for testing a diseased state of a subject based on a sample collected from the subject.

Background Art

In WO 03/012443 A2 there is described a rapid diagnostic device, assay and multifunctional buffer. A 2-step assay utilizes a dual component flow-through device comprising a test unit and a post-filter unit capable of receiving the fluid sample and multifunctional buffer, respectively. The test unit comprises a reaction zone containing immobilized capture reagent that can specifically bind to the target analyte, an absorbent zone supporting the reaction zone, and optionally, a blood separation zone in lateral fluid communication with the reaction zone. The post-filter unit comprises a label zone permeated with a dried indicator reagent which is capable of being placed in transient fluid communication with the reaction zone of the test unit during the assay procedure.

Further, Admin: "Rapid HIV Test by SD Bio-Line Kit", 29 January 2008, XP055101196 is related to a rapid HIV test by SD bio-line kit. Here, the presence of two color lines in a result window indicates a positive result for HIV-1. The presence of two color lines being different from the previous two color lines in the result window indicates a positive result for HIV-2. The presence of three color lines in the result window indicates a positive result for HIV-1 and HIV-2, and the presence of only one color line in the result window indicates a negative result.

Recently, a large number of assay devices that allow a simple and rapid testing have been developed in which a specimen (sample) which is likely to contain a test substance is held on a carrier, and the test substance is tested by immunological measurement or the like. Further, extracorporeal diagnostic agents and various devices for testing poisonous substances have also been commercially available. As an example of such devices, a device that uses an immunochromatographic method is known as described, for example, in Patent Document 1. Use of the device that utilizes the immunochromatographic method allows a test result to be obtained by holding a specimen solution and keeping the carrier stationary for 5 to 10 minutes, in the quickest case. For this reason, test methods that use assay methods, such as immunological testing and the like, are widely used, for example, in clinical inspection in hospitals and certification testing in laboratories, as simple and rapid test methods.

In particular, in medical care sites, such as doctor's office, clinic, home medical care, and the like, immunochromatographic test devices (immunochromato-reader) are widely used as test devices for POCT (Point of Care Testing) in which a simple test is performed by a person who is not a specialist of clinical inspection. The immunochromatographic test device may measure a color development state of a reagent loaded in the device with high sensitivity, thereby allowing high sensitivity and high reliability testing even when the color development state is so low that visual judgment is difficult. An example of such type of test device is described in Patent Document 2.

In the test method described above, it is demanded that a small amount of test substance is detected with high sensitivity. As a test method that responds to such demand, a method that performs amplification (sensitization) is known as described, for example, in Patent Document 3. In the method, a test substance is deployed on the carrier, then a cleaning solution is supplied to the carrier to clean the carrier other than a labeled substance captured by the reaction site on the carrier through specific binding, and a sensitizing solution is supplied to the carrier to effect sensitization, thereby allowing a small amount of test substance to be detected with high sensitivity.

Note that the sensitization may be performed on an as-required basis. That is, in the case where the color development state can be measured through the ordinary processing, the measurement is terminated, while if the color development state cannot be measured through the ordinary processing, the color development state may be measured after sensitization.

The aforementioned conventional test devices, such as the immunochromatographic test device, and the like, are often structured to perform testing using a reaction vessel having therein a carrier with a reagent held thereon. The reaction vessel is generally referred to as a cartridge, package, or test kit, and often configured to allow for a plurality of different diseases to be determined positive or negative by a plurality of different types of reagents.

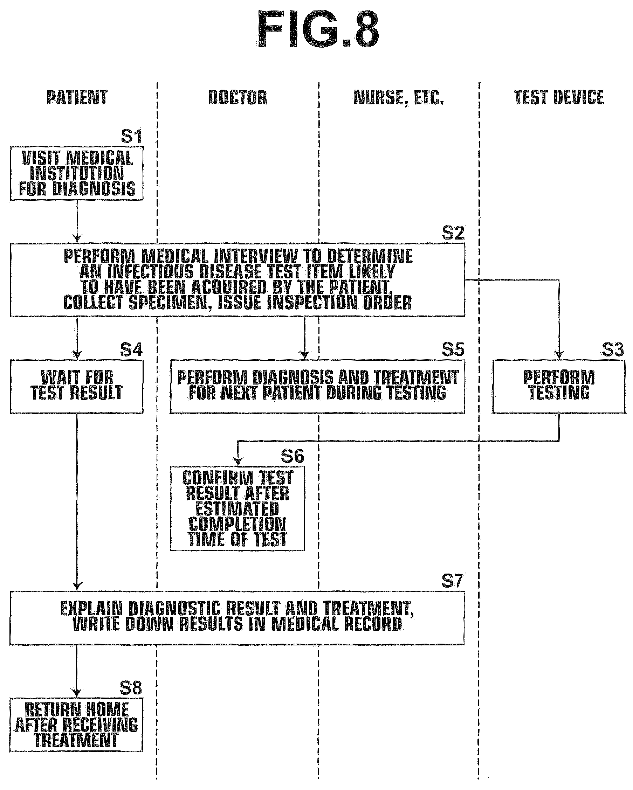

Now, Figure 8 illustrates conventional testing performed in a hospital or the like using the aforementioned immunochromatographic test device or the like and an associated processing flow. In Figure 8, steps to be performed by the patient, doctor, nurse or the like, and test device are demarcated by the vertical dotted lines. For example, a patient having a subjective symptom of an infectious disease, such as influenza, visits a medical institution, such as hospital, doctor's office, or the like (step S1 in Figure 8) . Then, a doctor, nurse, or the like performs a medical interview or the like to determine a test item for an infectious disease which is likely to have been acquired by the patient, then a specimen is collected from the patient and sent to an inspection center in the hospital or the like, and inspection order is issued (step S2) . In the inspection center or the like, a test for positive or negative judgment on the predetermined disease is performed using a test device (step S3).

The patient waits for a test result in a waiting lounge until the test is completed and a test result becomes available (step S4) . In the mean time, the doctor or the like performs diagnosis or treatment for the next patient (step S5).

Then, the doctor confirms the test result sent from the inspection center after the estimated completion time of the test (step S6). Thereafter, the doctor calls in the patient again and explains the diagnostic result and treatment based on the result, and writes down the diagnostic result and treatment in the medical record (step S7). The patient returns home after receiving the treatment (step S8).

Patent Document 4 describes that a test result is conveyed to a doctor by a communication means, and such conveying method may be applied to the test and diagnosis described above.

[Prior Art Documents]

[Patent Documents]

Patent Document 1:    Japanese Unexamined Patent Publication No. 2008-139297
Patent Document 2:    Japanese Unexamined Patent Publication No. 2009-133813
Patent Document 3:    Japanese Unexamined Patent Publication No. 2009-287952
Patent Document 4:    PCT Japanese Publication No.2008-518617

Disclosure of the Invention

The test and associated processing flow shown in Figure 8 are also applied to a plurality of diseases as described above. That is, positive or negative judgments are made by the test device on all of a plurality of diseases to be tested first and then test results are notified at a time to the doctor that has issued the inspection order.

But, this will result in that a patient needs to wait for a long time in the waiting lounge or the like before being called in by the doctor, and if the patient is affected and if, in particular, the disease is highly infectious, in-hospital infection may occur. Further, in the case where the patient is affected, an adverse situation may also arise in which the symptom is aggravated due to a prolonged time of waiting.

In view of the circumstances described above, it is an object of the present invention to provide, in a method for testing a patient for a plurality of diseases, a test method capable of preventing in-hospital infection and aggravation of the symptom of a patient arising from a prolonged stay in a medical institution.

A test method according to the present invention is a method having the features of claim 1, for testing with a test apparatus whether a subject is positive or negative for a plurality of diseases using a specimen collected from the subject,

wherein, if the subject is determined to be positive for one of the plurality of diseases, a test result indicating the positive is outputted from the test apparatus without waiting for completion of the testing of the other diseases.

Generally, the specimen is sent in spot applied to a test cartridge or the like from a person making an inspection request, such as the doctor or the like, to the operator of the test device, such as a medical technologist. In such a case, when the test result is outputted, it is preferable to generate a notice of test result to be sent to the person who has made the inspection request from the test apparatus. The term "notice of test result to be sent to the person who has made the inspection request" as used herein refers to all notices issued with the person who has made the inspection request as the destination. For example, a notice sent to a nurse working with the doctor, the person who has made the inspection request, via FAX or telephone and a printed notice with a note saying like "deliver this to doctor ○○ of △△ department" are included in such notices, as well as a notice sent to the person who has made the inspection request via the communication means described in Patent Document 4.

In the test method it is preferable that the testing for the one disease described above is performed first among the plurality of diseases to be tested.

Preferably, the testing for the one disease is arbitrarily selectable from the plurality of diseases to be tested.

Preferably, the test result indicating positive for the one disease is outputted by a means different from that for outputting test results of the other diseases. More specifically, if for example, the latter outputting means is FAX or telephone, the former output, the output indicating positive for the one disease may be via an electronic mail system, or vice versa. Further, if the means for implementing the latter output is a white facsimile paper, then the means for implementing the former output may be a colored facsimile paper, or vice versa.

More preferably, the test method of the present invention is applied to the case in which the disease to be tested is an infectious disease.

Preferably, the test method of the present invention is applied to the case in which a plurality of diseases whose therapeutic agents are the same is set as the test targets.

In the meantime, the test apparatus is an apparatus having the features of claim 7, configured to allow a subject to be tested positive or negative for a plurality of diseases using a specimen collected from the subject, wherein, the apparatus includes a means for outputting, if the subject is determined to be positive for one of the plurality of diseases, a test result indicating the positive without waiting for completion of the testing of the other diseases.

According to the test method of the present invention, if a subject is determined to be positive for one of a plurality of diseases, a test result indicating the positive is outputted from the test apparatus without waiting for completion of the testing of the other diseases. This allows the person who has made the inspection request, such as the doctor, to know a positive test result for a particular disease, for example, highly infectious disease or the like, for the time being before all of the tests are completed after a long time. Then, the doctor or the like may immediately call in the patient waiting in the waiting lounge of a medical institution for treatment only for the disease and the patient may return home after that. This may prevent a prolonged stay of the patient in the hospital or the like, whereby in-hospital infection or aggravation of the symptom of the patient may be prevented.

In the case where a notice of test result to be sent to the person who has made the inspection request is generated from the test apparatus, in particular, the test result may be delivered to the person who has made the inspection request in a shorter period of time.

Further, if the one disease described above is a disease to be tested first among a plurality of diseases, in particular, the time for the patient to stay in the hospital or the like may be minimized, so that the advantageous effects of preventing in-hospital infection or prevention of symptom aggravation of the patient become significant.

In the test method in particular, if the testing for the one disease described above is made arbitrarily selectable from a plurality of diseases to be tested, then, the in-hospital infection may be prevented more reliably by, for example, making an appropriate response in which the testing for the influenza of increased infectiousness is completed sooner according to the occurrence of influenza in each year.

If the test method of the present invention is applied to the case where the test target is an infectious disease, such as influenza or the like, in-hospital infection of the disease may be prevented as described above. In the case where the test target is not an infectious disease also, the application of the present invention may provide advantageous effects of preventing symptom aggravation of the patient due to prolonged stay in the hospital or the like for a test result.

If the test method of the present invention is applied to the case where a plurality of diseases is those whose therapeutic agents are the same, in particular, a test result is outputted at the time when one test result is determined to be positive and the test result is notified to the doctor, so that the doctor may prescribe an appropriate therapeutic agent without exactly knowing what kind of the disease it is which has been determined to be positive.

In the mean time, the test apparatus includes a means for outputting, if the subject is determined to be positive for one of the plurality of diseases, a test result indicating the positive without waiting for completion of the testing of the other diseases, as described above. Thus, the test method of the present invention described above may be implemented with the apparatus.

Brief Description of Drawings

Figure 1 is a perspective view of an immunochromatographic test device.
Figure 2 is a partially broken side view of the test device described above.
Figure 3 is a block diagram, illustrating electrical configuration of the test device described above.
Figure 4 is a front elevation view illustrating a portion of the test device.
Figure 5 is a partially broken plan view of a cartridge used in the test device, illustrating a certain state thereof.
Figure 6 is a partially broken plan view of the cartridge, illustrating another state thereof.
Figure 7 is a partially broken plan view of the cartridge, illustrating a still another state thereof.
Figure 8 is a flowchart illustrating a processing flow in a conventional test method.
Figure 9 is a flowchart illustrating a testing flow in the test device described above.

Best Mode for Carrying Out the Invention

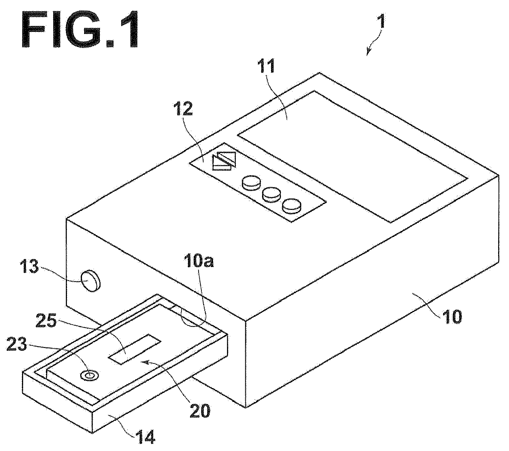

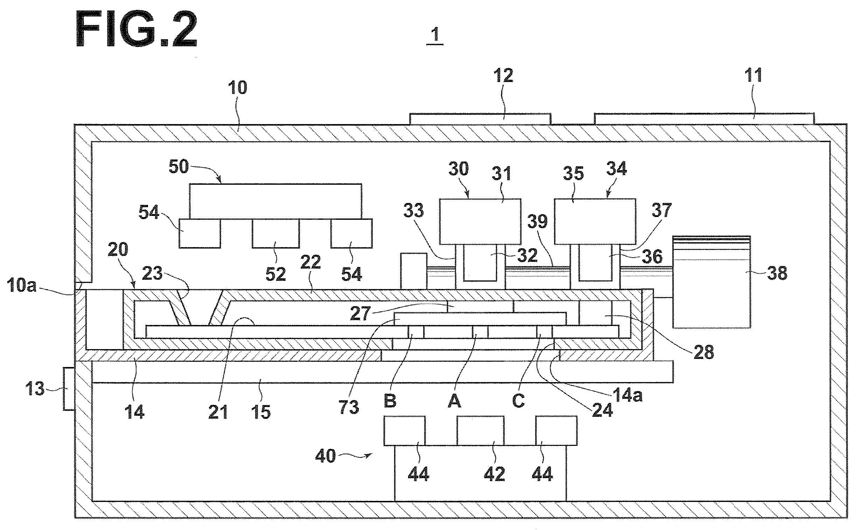

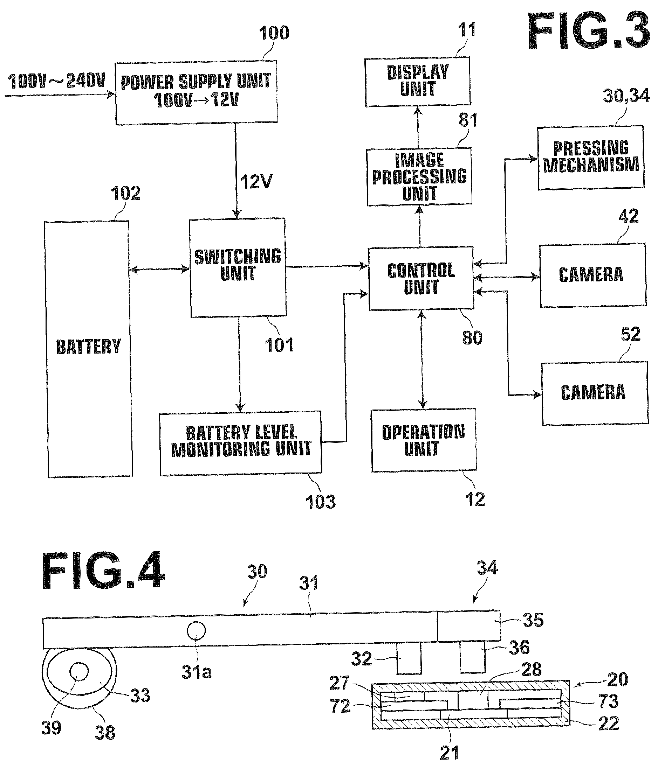

Hereinafter, embodiments of the present invention will be described in detail with reference to the accompanying drawings. Figure 1 illustrates a perspective view of an immunochromatographic test device 1 according to an embodiment of the present invention, Figure 2 illustrates partially broken side view thereof, and Figure 3 illustrates an electrical configuration thereof. A basic configuration of the device will be described first with reference to Figures 1 and 2.

As illustrated in these drawings, the immunochromatographic test device 1 includes a housing 10 having an opening 10a in front, a display unit 11 disposed on the upper surface of the housing 10, an operation unit 12 for operating a menu displayed on the display unit 11, a power switch 13, and a cartridge loading unit 14 for loading an immunochromatographic cartridge 20 inside of the device. The device 1 further includes, inside of the housing 10, a rail 15 for movably guiding the cartridge loading unit 14 in left-right directions in Figure 2, pressing units 30, 34 for crushing a cleaning solution pot 27 and sensitizing solution pot 28 (to be described later) respectively, and first and second measurement units 40 and 50 for obtaining information from the cartridge 20.

The cartridge loading unit 14 is movable automatically or manually along the rail 15. When a most part of the unit is drawn out of the housing 10 past the opening 10a, the cartridge 20 supplied with a test solution in a manner to be described later is placed thereon. Then the cartridge loading unit 14 is pushed into the housing 10, as illustrated in Figure 2, and thereby the cartridge 20 is brought into inside of the immunochromatographic test device 1.

Figure 4 is a front elevation view of portions of the pressing units 30, 34 viewed from the left side in Figure 2. Note that the cartridge 20 is illustrated in broken form. The pressing units 30, 34 will now be described with reference to Figure 4. The pressing unit 30 includes an arm 31 rotatable around a shaft 31a, a pressing piece 32 fixed to the underneath of the tip of the arm 31, and a cam 33 disposed under the back end of the arm 31. The cam 33 is disconnectably connected to a drive shaft 39 to be rotated by a motor 38 via an electromagnetic clutch or the like (not shown). When the cam 33 is rotated, the back end of the arm 31 is pushed up and the pressing piece 32 at the tip is moved downward. Likewise, the other pressing unit, that is the pressing unit 34, includes an arm 35, a pressing piece 36, and a cam 37, and is structured in the same manner as that of the pressing unit 30.

The pressing piece 32 of the pressing unit 30 and the pressing piece 36 of the pressing unit 34 are arranged so as to locate immediately above the cleaning solution pot 27 and sensitizing solution pot 28 disposed inside of the kit respectively when the cartridge 20 is placed at a given position in the housing 10.

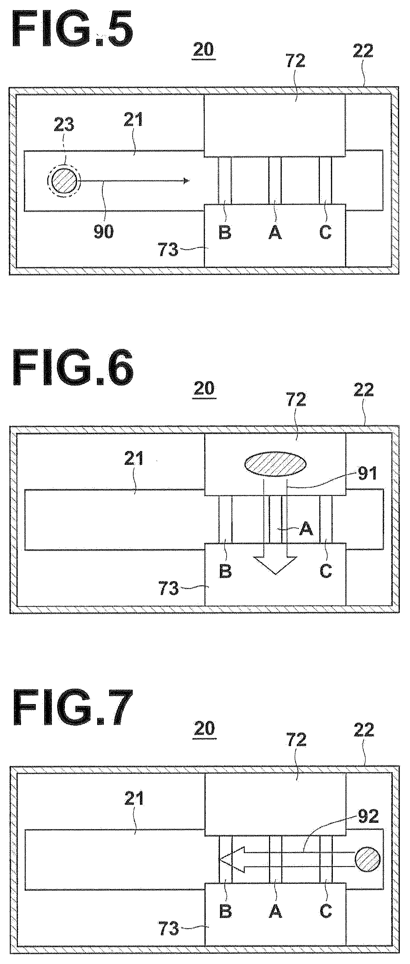

Figure 5 is a broken plan view of the cartridge 20 broken at the upper surface. Hereinafter, the cartridge 20 will be described with reference also to Figure 5. The cartridge 20 includes an insoluble carrier 21 having a test line A that carries a reagent in a strip-like manner, a test line B that carries a different reagent from that described above in a strip-like manner, and a strip-shaped control line C, a kit case 22 housing the insoluble carrier 21, a solution injection opening 23 formed in the upper surface of the case to inject a specimen solution, and an observation window 24 for observing a test region (portions of the test lines A, B and control line C). An information display section 25 is provided on the upper surface of the kit case 22. Note that an observation window 14a substantially corresponding to the observation window 24 is provided on the cartridge loading unit 14.

The insoluble carrier 21 has an immobilized labeling substance. Each of the test lines A, B has a specific binding substance for a specimen (test substance) immobilized thereon as a reagent and the control line C is provided to determine the end of the measurement.

Further a solution sending insoluble carrier 72 and an absorption insoluble carrier 73 are arranged inside of the cartridge 20 so as to sandwich the insoluble carrier 21. The cleaning solution pot 27 is fixed above the solution sending insoluble carrier 72 and the sensitizing solution pot 28 is fixed above the end portion of the insoluble carrier 21 on the control line C side. The upper surface portion of the case 22 of the cartridge 20 deforms easily when pressed down from above by the pressing pieces 32 and 36 described above to allow the cleaning solution pot 27 and sensitizing solution pot 28 to be crushed.

The first measurement unit 40 measures a color development state of the test region (portions of the test lines A, B and control line C) through the observation window 24 of the cartridge 20. As illustrated in Figure 2, the first measurement unit 40 includes a camera 42 and a light source 44 which are arranged to face the observation window 24 from below the cartridge 20 when the cartridge 20 is loaded in the test device 1. Then, based on the optical information of the test region obtained by the first measurement unit 40, optical density and chromaticity are calculated as the color development state of the test region (to be described later).

The optical density is defined by the formula given below when the intensity of the incident light incident on the test region of the cartridge 20 is taken as I and the intensity of the reflection light from the test region is taken as Ir:

$$\texttt{Optical density = -log10(Ir/I)}$$

The chromaticity is a quantitative representation of hue and saturation and is calculated from the RGB luminance signal captured by the camera. As for the color system of the chromaticity, the general CIE color system may be used.

The camera 42 includes, for example, a plurality of photodiodes arranged in a line or an image sensor formed of an area sensor, and generates an output according to the amount of light received. The light receiving range of the camera 42 is set to the strip-like range extending in the longitudinal direction of the cartridge 20. The light source 44 is, for example, a module having an LED therein and configured to emit white light. The light source 44 may be, for example, a monochromatic light source if it allows distinction between chromaticity values before and after sensitization, to be described later. In the case where the light source 44 includes a plurality of modules, each module may emit monochromatic light of a different wavelength. The light emitted from the light source 44 is set to reach a predetermined range in the longitudinal direction of the cartridge 20.

In the mean time, the second measurement unit 50 directs illumination light to the information display section 25 of the cartridge 20 and obtains information displayed on the information display section 25. The information display section 25 is a place where information related to the test is displayed by handwriting, attaching a seal, or the like. The information related to the test may include, for example, information of the patient from which the test substance has been collected (name, age, gender, and the like) and information of sample and reagent used in the testing (test target specimen, names of the cleaning solution and sensitizing solution used, and the like). There is not any specific restriction on the method of obtaining the information, and the information display section 25 may be imaged directly or information may be read from bar coded information.

As illustrated in Figure 2, the second measurement unit 50 includes a camera 52 and a light source 54 which are arranged to face the information display section 25 from above the cartridge 20 when the cartridge 20 is loaded in the test device 1. The information related to the test obtained by the second measurement unit 50 and test results are related to each other for management. The specific configurations of the camera 52 and light source 54 are identical to those of the camera 42 and light source 44 respectively.

The electrical configuration of the device will now be described with reference to Figure 3. The already described display unit 11, operation unit 12, pressing mechanism 30, 34 which includes the motor 38 and the like, cameras 42, 52 (each including light source 44, 54 respectively) are controlled in operation by a control unit 80. The test device 1 can be operated on commercial power with a voltage, for example, of 100 to 240 V, and includes a power supply unit 100 for receiving the commercial power and transforming it into a 12V DC current and a switching unit 101 to which the 12V DC current is inputted. In addition, the test device 1 can be operated also on a secondary cell battery 102 and the battery 102 is also connected to the switching unit 101. The switching unit 101 performs switching such that a 12V DC current supplied from the power supply unit 100 is used by each electric component if the commercial power is connected while if the commercial power is not connected, a 12V DC current supplied from the battery 102 is used.

Further, a battery level monitoring unit 103 as a remaining battery level detection means for detecting a remaining amount of power in the battery 102 is connected to the switching unit 101. Generally, a battery has a property that the internal resistance increases as the battery level is reduced due to its chemical characteristics and the terminal voltage is reduced. Thus, the amount of remaining power in the battery can be detected by measuring the terminal voltage. In this way, the battery level monitoring unit 103 keeps monitoring the amount of remaining power in the battery 102 and a signal representing the amount of remaining power is inputted to the control unit 80.

Next, measurement performed by the test device 1 will be described. In principle, the present device performs a first stage measurement followed by a second stage measurement. As the first stage measurement, a color development state of the test region is measured without sensitization, to be described later, and the color development state of the test region is measured after the sensitization, as the second stage measurement.

First, a specific operational procedure for the measurement will be described.

[First Stage Measurement]

In the first stage measurement, for example, a specimen solution 90 is injected into the cartridge 20 from the solution injection opening 23 outside of the test device 1, as illustrated in Figure 5. Thereafter, the cartridge 20 is loaded inside of the test device 1 in the manner described above and the test region (portions of the test line A, test line B, and control line C) of the cartridge 20 is imaged by the camera 42 in order to calculate the optical density and chromaticity of the region. The control unit 80 shown in Figure 3 causes a two-dimensional image signal outputted from the camera 42 to be inputted to the image processing unit 81.

The image processing unit 81 calculates a color development state of reagent portion, i.e., optical density and chromaticity of the portion based on the two-dimensional signal and displays the calculated values together with a positive or negative judgment for disease made based on the values on the display unit 11 as the detection results.

[Second Stage Measurement]

In the second stage measurement, the pressing unit 30 shown in Figures 2 and 4 is driven to move the tip of the arm 31 downward whereby the pressing piece 32 crushes the cleaning solution pot 27 located in the cartridge 20 from the outside. This causes the test region of the insoluble carrier 21 to be cleaned by a cleaning solution 91 stored in the cleaning solution pot 27, as illustrated in Figure 6. Here, the cleaning solution 91 spreads sufficiently in the solution sending insoluble carrier 72 first, and then moves to the insoluble carrier 21 and absorption insoluble carrier 73 successively.

Next, the pressing unit 34 shown in Figures 2 and 4 is driven to move the tip of the arm 35 downward whereby the pressing piece 36 crushes the sensitizing solution pot 28 located in the cartridge 20 from the outside. This causes a sensitizing solution 92 to be sent to the test region of the insoluble carrier 21, whereby sensitization is performed. The sensitizing solution 92 and cleaning solution 91 are described in detail in Patent Documents 3 and those described in the document may be applied.

After the sensitization, the test region of the cartridge 20 is imaged by the camera 42 in the manner described above. The control unit 80 shown in Figure 3 causes a two-dimensional image signal outputted from the camera 42 to be inputted to the image processing unit 81. The image processing unit 81 performs the same processing on the two-dimensional image signal as that performed in the first stage measurement. In this case also, a color development state of reagent portion, i.e., optical density and chromaticity values of the portion and a positive or negative judgment for disease made based on the values are displayed on the display unit 11 as the detection results.

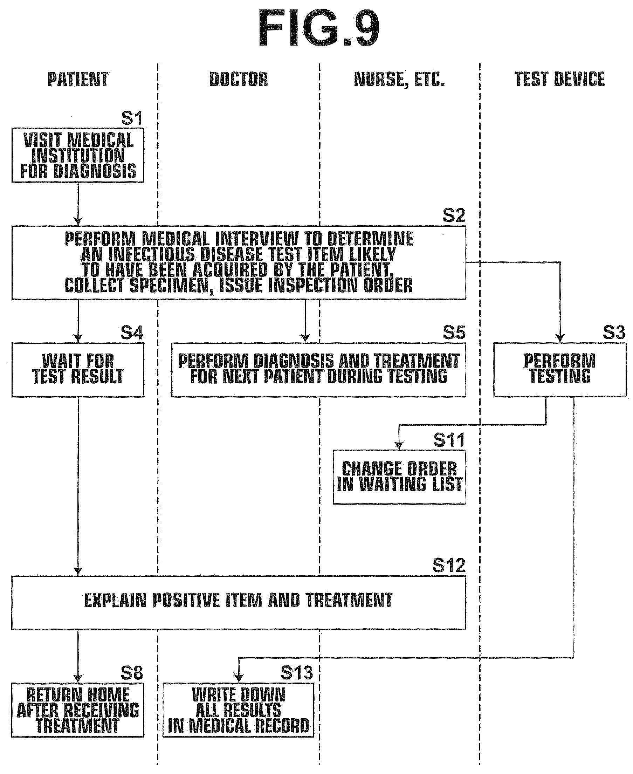

Associated processing performed in the hospital or the like before and after the testing performed in the manner described above will now be described with reference to Figure 9 that illustrates the processing flow. Figure 9 illustrates processing steps performed from the time when a patient visits a hospital to the time when the patient returns home after receiving a treatment, in which steps S1 to S5 are identical to those shown in Figure 8.

The testing described above is shown in Figure 9 as a step S3 and further described in detail. In the present example, the test lines A, B are used for testing positive or negative for different infectious diseases "a" and "b", such as influenza and the like. In either case in which only the first stage measurement is performed or in which the second stage measurement is performed in addition to the first stage measurement, the positive testing for the infectious disease "a" by calculating the optical density and chromaticity of the colored test line A and positive testing for the infectious disease "b" by calculating the optical density and chromaticity of the colored test line B are performed on an as-required basis, for example, at a time interval of about one minute during reaction of the reagents. When the optical density of the respective test lines A, B exceeds a predetermined level, the corresponding test item is determined to be positive. A test item for which no color reaction appears after a maximum relation time is determined to be negative. Taking an influenza test, as an example, the maximum reaction time is about 15 minutes while the positive judgment is often made three to five minutes after the start of the testing.

Normally, therefore, the positive test result is obtained before the negative test result, and if a test result of either one of the test lines (for example, test line A) is positive for the infectious disease "a", the image processing unit 81 shown in Figure 3 immediately displays a message on the display unit 11 like, for example, "infectious disease "a": positive, inform this to doctor ○○ of △△ department" without waiting for completion of the test by the other test line (for example, test line B). The medical technologist took note of the display makes a phone call to a nurse working with the doctor who has issued the test order request (doctor ○○) to inform that the test result for the infectious disease "a" is positive together with the patient information. Note that the display unit 11 constitutes the means for issuing an output representing positive.

The nurse received the information or the like changes the order in waiting list by changing medical cards (step S11) so that the patient, the subject of the testing described above, will be called in by the doctor next to the patient currently being examined or treated by the doctor. Then, the doctor explains the positive item and treatment for the item (step S12) and performs the treatment. The patient returns home after receiving the treatment (step S8).

Thereafter, all of the test results including a positive or negative judgment for the infectious disease "b" are delivered to the doctor from the medical technologist who performed the testing, and the doctor writes down the contents of the aforementioned treatment and all of the test results in the medical record (step S13), thereby completing a series of diagnosis for the patient.

As described above, if positive is confirmed for an infectious disease "a", an output representing the positive is generated and the output is informed to the doctor who have made an inspection request without waiting for completion of the test for the other infectious disease "b". This allows the doctor to know the positive test result for the infectious disease "a" for the time being before all of the tests are completed after a long time. Then, the doctor may return the patient home early after the explanation and treatment described above. This may prevent a prolonged stay of the patient in the hospital or the like, whereby in-hospital infection or aggravation of the symptom of the patient may be prevented.

The test method of the present invention may perform positive or negative testing for a plurality of diseases. There may be a case in which the same therapeutic agent may be used even when any of the plurality of diseases becomes positive. For example, in the case of type A influenza and type B influenza testing, the same antiviral medicine is administered in either case in which the type A influenza or type B influenza is positive. In such a case, if a test result is outputted at the time when one test result is determined to be positive, the doctor informed of the test result may prescribe an appropriate therapeutic agent without exactly knowing what kind of the disease it is which has been determined to be positive.

In the present example, testing for two types of diseases is performed, but the present method is applicable to the case in which positive or negative detection is performed for three or more diseases. In such a case, the test whose result needs to be preferentially informed to the person who has made the inspection request may be any test other than that performed last in any event. But, if the test whose result needs to be preferentially informed to the person who has made the inspection request is arranged to be performed first, the time for the patient to stay in the hospital or the like may be reduced the most if the test result thereof is positive, so that advantageous effects of the prevention of in-hospital infection or aggravation of the symptom of the patient become significant.

In particular, in the case where the test whose result needs to be preferentially informed is made arbitrarily selectable from a plurality of tests in the test method of the present invention, then, the in-hospital infection may be prevented more reliably by, for example, making an appropriate response in which the test for the influenza of increased infectiousness is completed sooner according to the occurrence of influenza in each year.

In the case where the display unit 11 shown in Figure 3 includes a means for outputting a recording paper, the aforementioned message "infectious disease "a": positive, inform this to doctor ○○ of △△ department" may be displayed and the same contents may be recorded on the recording paper and outputted at the same time. In such a case, it is particularly preferable that the aforementioned output is recorded on a color paper, unlike the normal case in which test results are outputted on a white paper, since the recording is clearly indicated to be sent immediately to the person who has made the inspection request.

Further, if an arrangement is adopted in which a sound alarm is issued when the positive test result is obtained for the infectious disease "a" in the manner described above, the medical technologist or the like may be informed reliably that a test result which should be sent to the person who has made the inspection request is obtained.

Items associated with the aforementioned measurement will be described briefly.

(Specimen Solution)

There is not any specific restriction on the specimen solutions which can be analyzed by the test device as long as they are likely to include test substances (natural products, poisons, biologically active agents such as hormones, agricultural chemicals, and the like, or environmental pollutants, and the like). For example, biological samples, in particular, animal (in particular, human) body fluids (e.g., bloods, serums, blood plasmas, spinal fluids, tear fluids, sweats, urines, purulent matters, snivels, or sputum), or body wastes (e.g., faces), organs, tissues, mucous membranes, skins, or scratched specimens (swabs) believed to include these, gargled solutions, animals or plants themselves, or dried bodies thereof diluted by a diluting fluid, to be described later.

The specimen solution may be used directly, in the form of extraction liquid extracted using an appropriate extraction solvent, in the form of diluted solution obtained by diluting the extraction liquid with an appropriate diluting agent, or in the condensed form of the extraction liquid condensed by an appropriate method.

(Labeling Substance)

There is not any specific restriction on the labeling substance that can be used as long as it can be visually recognizable or detectable through reaction, such as metal fine particles (or metallic colloids) used in general immunochromatographic methods, colored latex particles, enzymes, and the like. In the case where a signal is sensitized through deposition of metal on the labeling substance due to reduction reaction of metal ions with the labeling substance as the catalyst, however, metal particles are preferably used in view of the catalytic activity.

As for the material of the metal fine particles, single metal body, metallic sulfide, metal alloy, or polymer particle that includes the metal may be used. Preferably, the average particle diameter of the particles (colloids) is in the range of 1 nm to 10 μm. The average particle diameter as used herein refers to an average value of diameters (longest diameters) of a plurality of particles measured by a transmission electron microscope (TEM). More specifically, gold colloids, silver colloids, platinum colloids, iron colloids, aluminum hydroxide colloids, and complex colloids of these may be cited, and gold colloids, silver colloids, platinum colloids, and complex colloids of these are preferably used. Among them, the gold colloids and silver colloids are particularly preferable in view of the fact that the gold colloids and silver colloids having an appropriate particle diameter appear in red and yellow respectively, thereby providing high visibility. The use of gold colloids and performance of sensitization process using a silver-ion containing compound cause the chromaticity of the label to be changed before and after the sensitization process (the gold colloids are colored red which turn to black after the sensitization due to deposition of reduced silver ions on the gold colloids) . Thus, this change may be used for the judgment of a test error as described later. Preferably, the average particle diameter of the metal colloids is 1 to 500 nm and more preferably, 1 to 100 nm.

(Specific Binding Substance)

There is not any specific restriction on the specific binding substance as long as it has affinity to the test substance. For example, if the test substance is an antigen, the specific binding substance may be the antibody to the antigen, if the test substance is protein or a metal ion or low molecular organic compound, the specific binding substance may be an aptamer to these, if the test substance is a nucleic acid, such as DNA or RNA, the specific binding substance may be a nucleic acid molecule such as DNA or RNA having a complementary sequence to these, if the test substance is an avidin, the specific binding substance may be a biotin, and if the test substance is a particular peptide, the specific binding substance may be a complex that specifically bind to the peptide. Further, the relationship between the specific binding substances and test substances described above may be replaced and, for example, if the test substance is an antibody, the antigen to the antibody may be used as the specific binding substance. Further, compounds which partially include substances having affinity to those test substances described above may be used as the specific binding substances.

As for the antibody describes above, an antiserum prepared from an animal serum immunized by the test substance, an immunoglobulin faction purified from the antiserum, a monoclonal antibody obtained by cell fusion using an animal spleen cell immunized by the test substance, or fragments of these (such as F(ab')2, Fab, Fab', or Fv) may specifically used. These antibodies may be prepared by an ordinary method.

(Insoluble Carrier)

Preferably, the material of the insoluble carrier 21 is porous and, for example, nitrocellulose membranes, cellulose membranes, cellulose acetate membranes, polysulfone membranes, polyether sulfone membranes, nylon membranes, glass fibers, nonwoven fabrics, fabrics, threads, or the like are preferably used.

A test line is created on a chromatograph carrier by immobilizing a specific binding substance for a test substance with a control region as required. The specific binding substance may be directly immobilized on a portion of the chromatograph carrier physically or through chemical bonding. Alternatively, the specific binding substance may be immobilized on fine particles, such as latex particles or the like, physically or through chemical bonding, and the particles may be immobilized on a portion of the chromatograph carrier by trapping.

(Sensitizing Solution)

The sensitizing solution is a solution that produces a compound that develops a color or emits light through a reaction of the chemical agent included therein by way of catalytic action of the labeling substance or test substance, thereby capable of sensitizing a signal. For example, it may be a silver ion solution that causes deposition of metallic silver on a metallic labelling due to physical development. More specifically, so-called developers may be used as described in general books in the field of photographic chemicals (for example, "Revised Basic Photographic Engineering - Silver Halide Photography", Edited by Society of Photographic Science and Technology of Japan, Corona Publishing, Co., Ltd., "Chemicals of Photography", Akira Sasai, Photography Industry Publishing Co., Ltd. and "Handbook of Recent Prescription", Shinich Kikuchi et al., AMIKO Publishing) . For example, the use of a physical developer which includes a compound having silver ions as the sensitizing solution may reduce silver ions in the solution around metal colloids serving as cores of development or the like by the reducing agent of the silver ions.

Another example is to use enzyme reaction. For example, a solution of phenylenediamine compound and naphthol compound which becomes a dye by the action between a peroxidase label and hydrogen peroxide may be used. Further, a chromogenic substrate used in horseradish peroxidase detection as described in a non-patent document "Staining Utilizing H2O2-POD System", Clinical Examination, Vol. 41, No. 9, pp. 1020-1024, may also be used. The chromogenic substrate described in Japanese Unexamined Patent Publication No. 2009-156612 is particularly preferable. Still further, a system utilizing a metal catalyst, such as platinum fine particles, instead of enzyme may also be used.

As for another example that utilizes a different enzyme, a system that develops a color with alkaline phosphatase as the labelling and 5-bromo-4-chloro-3-indolyl phosphate disodium salt (BCIP) as the substrate is known. So far, chromogenic reactions have been described as representative examples, but any combination of enzyme and substrate may be used, in which the substrate may be that which emits chemiluminescence or fluorescence.

(Silver Ion Containing Compound)

As for the silver ion containing compound, an organic silver salt, inorganic silver salt, or silver complex may be used. Silver ion containing compounds having high solubility in solvent, such as water, are preferable, and such compounds includes silver nitrate, silver acetate, silver lactate, butanoic acid silver, silver thiosulfate, and the like. Among them, silver nitrate is particularly preferable. As for the silver complex, a silver complex coordinated with a ligand having a water-soluble group such as a hydroxyl group or a sulfone group is preferable. An example of such a silver complex may be hydroxy thioether silver or the like. Preferably, the inorganic silver salt or silver complex is included as silver generally in the amount of 0.001 mole/m$^2$ to 0.2 mole/m$^2$, and more preferably 0.01 mole/m$^2$ to 0.05 mole/m$^2$.

(Reducing Agent for Silver Ions)

As for the reducing agent used for silver ions, any material, such as an inorganic material, organic material, or a mixture thereof, can be used, as long as it can reduce silver ions to silver.

Preferable examples of inorganic reducing agent include reducing metal salts and reducing metal complex salts whose valence can be changed with metal ions such as $Fe^{2+}$, $V^{2+}$, or $Ti^{3+}$. In the case where an inorganic reducing agent is used, it is necessary to remove or render harmless oxidized ions through complexation or reduction. For example, in a system that uses $Fe^{2+}$ as the reducing agent, a complex of $Fe^{3+}$, as an oxide, is formed with citric acid or EDTA, whereby the oxidized ions can be rendered harmless. In the present system, it is preferable to use such an inorganic reducing agent, and metal salt of $Fe^{2+}$ is more preferably used.

In the example described above, a method in which labeling substance is sensitized by reducing a silver ion containing compound by a reducing agent as the sensitizing method for color development, but the sensitizing method is not limited to this. The sensitizing solution may be any solution as long as it is capable of producing a compound that develops a color or emits light through a reaction of the chemical agent included therein by way of catalytic action of the labeling substance or test substance and sensitizing a signal. An example may be a solution of the enzyme described above.

In the example described above, the immunochromatographic method has been described as an assay method, but the assay method is not limited to this. It is applicable to a system that does not use so-called immunoreactions. For example, the present methods may be applied to a system in which a test substance is captured by a nucleic acid, such as DNA or RNA without using the antibody or a system in which a test substance is captured by a different small molecule having affinity to the test substance, peptide, protein, complex forming substance, or the like.

## Claims

1. A test method for testing with a test apparatus (1) whether a subject is positive or negative for a plurality of diseases using a specimen collected from the subject,
   ***characterized in* that**
   if the subject is determined to be positive for one of the plurality of diseases, a test result indicating the positive is outputted from the test apparatus (1) without waiting for completion of the testing of the other diseases; and
   the test result indicating the positive is sent as notice of test result from the test apparatus (1) via a first communication means to a user who requested inspection.

2. The test method of claim 1, wherein the first communication means is different from a second communication means for outputting test results of the other diseases.

3. The test method of claim 1, wherein the first communication means is an electronic mail system and the second communication means is FAX or telephone, or vice versa.

4. The test method of claim 1, wherein the testing for the one disease is arbitrarily selectable from the plurality of diseases to be tested.

5. The test method of any of claims 1 to 4, wherein the disease to be tested is an infectious disease.

6. The test method of any of claims 1 to 5, wherein the plurality of diseases is those whose therapeutic agents are the same.

7. Use of a test apparatus (1) configured to allow a subject to be tested positive or negative for a plurality of diseases using a specimen collected from the subject according to the method of any one of the claims 1 to 6.

## Patentansprüche

1. Ein Testverfahren zum Testen mit einer Testvorrichtung (1), ob eine Testperson positiv oder negativ bezüglich einer Vielzahl von Krankheiten ist, unter Verwendung einer von der Testperson genommenen Probe,
   **dadurch gekennzeichnet, dass**,
   falls die Testperson als positiv hinsichtlich einer der Vielzahl von Krankheiten bestimmt ist, ein Positiv angebendes Testergebnis von der Testvorrichtung (1) ausgegeben wird, ohne auf den Abschluss des Testens der anderen Krankheiten zu warten; und
   das Positiv angebende Testergebnis als eine Benachrichtigung über das Testergebnis von der Testvorrichtung (1) über erste Kommunikationsmittel an einen Anwender gesendet wird, welcher die Überprüfung angefordert hat.

**2.** Das Testverfahren gemäß Anspruch 1, wobei die ersten Kommunikationsmittel sich von zweiten Kommunikationsmitteln zum Ausgeben von Testergebnissen der anderen Krankheiten unterscheiden.

**3.** Das Testverfahren gemäß Anspruch 1, wobei die ersten Kommunikationsmittel ein elektronisches Postsystem sind und die zweiten Kommunikationsmittel ein Fax oder Telefon sind, oder umgekehrt.

**4.** Das Testverfahren gemäß Anspruch 1, wobei das Testen für die eine Krankheit beliebig aus der Vielzahl von zu testenden Krankheiten auswählbar ist.

**5.** Das Testverfahren gemäß einem der Ansprüche 1 bis 4, wobei die zu testende Krankheit eine ansteckende Krankheit ist.

**6.** Das Testverfahren gemäß einem der Ansprüche 1 bis 5, wobei die Vielzahl von Krankheiten diejenige ist, deren Therapeutika identisch sind.

**7.** Verwendung einer Testvorrichtung (1), ausgebildet zum Ermöglichen, dass eine Testperson positiv oder negativ für eine Vielzahl von Krankheiten getestet wird, unter Verwendung einer von der Testperson genommenen Probe gemäß dem Verfahren gemäß einem der Ansprüche 1 bis 6.

**Revendications**

**1.** Procédé de test pour tester avec un appareil de test (1) si un sujet est positif ou négatif pour une pluralité de maladies en utilisant un échantillon prélevé sur ledit sujet,
**caractérisé en ce que**,
si le sujet est déterminé comme étant positif pour l'une de la pluralité de maladies, un résultat de test indiquant le positif est émis par l'appareil de test (1) sans attendre l'achèvement du test pour les autres maladies ; et
le résultat de test indiquant le positif est envoyé comme avis du résultat de test de l'appareil de test (1) via un premier moyen de communication à un utilisateur ayant demandé l'inspection.

**2.** Procédé de test selon la revendication 1, dans lequel le premier moyen de communication est différent d'un second moyen de communication pour délivrer des résultats de test des autres maladies.

**3.** Procédé de test selon la revendication 1, dans lequel le premier moyen de communication est un système de courrier électronique et le second moyen de communication est un fax ou le téléphone, ou inversement.

**4.** Procédé de test selon la revendication 1, dans lequel le test pour une maladie peut être arbitrairement sélectionné parmi la pluralité de maladies à tester.

**5.** Procédé de test selon l'une quelconque des revendications 1 à 4, dans lequel la maladie à tester est une maladie infectieuse.

**6.** Procédé de test selon l'une quelconque des revendications 1 à 5, dans lequel la pluralité de maladies est constituée de celles dont les agents thérapeutiques sont les mêmes.

**7.** Utilisation d'un appareil de test (1) configuré pour permettre à un sujet d'être testé positif ou négatif pour une pluralité de maladies en utilisant un échantillon prélevé sur le sujet conformément au procédé selon l'une quelconque des revendications 1 à 6.

# FIG.1

# FIG.2

# FIG.3

100V~240V → POWER SUPPLY UNIT 100V→12V — 100

DISPLAY UNIT — 11

IMAGE PROCESSING UNIT — 81

PRESSING MECHANISM — 30,34

12V

BATTERY — 102

SWITCHING UNIT — 101

CONTROL UNIT — 80

CAMERA — 42

CAMERA — 52

BATTERY LEVEL MONITORING UNIT — 103

OPERATION UNIT — 12

# FIG.4

30  31  34  35  36  32  27  72  28  20  73  22  21  31a  33  39  38

# FIG.5

20    72    22

23    21

B    A    C

90    73

# FIG.6

20    72    22

21

91

A

B    C

73

# FIG.7

20    72    22

21

92

B    A    C

73

# FIG.8

| PATIENT | DOCTOR | NURSE, ETC. | TEST DEVICE |
|---|---|---|---|

**S1**
VISIT MEDICAL INSTITUTION FOR DIAGNOSIS

**S2**
PERFORM MEDICAL INTERVIEW TO DETERMINE AN INFECTIOUS DISEASE TEST ITEM LIKELY TO HAVE BEEN ACQUIRED BY THE PATIENT, COLLECT SPECIMEN, ISSUE INSPECTION ORDER

**S4**
WAIT FOR TEST RESULT

**S5**
PERFORM DIAGNOSIS AND TREATMENT FOR NEXT PATIENT DURING TESTING

**S3**
PERFORM TESTING

**S6**
CONFIRM TEST RESULT AFTER ESTIMATED COMPLETION TIME OF TEST

**S7**
EXPLAIN DIAGNOSTIC RESULT AND TREATMENT, WRITE DOWN RESULTS IN MEDICAL RECORD

**S8**
RETURN HOME AFTER RECEIVING TREATMENT

# FIG.9

| PATIENT | DOCTOR | NURSE, ETC. | TEST DEVICE |
|---|---|---|---|

**S1**
VISIT MEDICAL INSTITUTION FOR DIAGNOSIS

**S2**
PERFORM MEDICAL INTERVIEW TO DETERMINE AN INFECTIOUS DISEASE TEST ITEM LIKELY TO HAVE BEEN ACQUIRED BY THE PATIENT, COLLECT SPECIMEN, ISSUE INSPECTION ORDER

**S4**
WAIT FOR TEST RESULT

**S5**
PERFORM DIAGNOSIS AND TREATMENT FOR NEXT PATIENT DURING TESTING

**S3**
PERFORM TESTING

**S11**
CHANGE ORDER IN WAITING LIST

**S12**
EXPLAIN POSITIVE ITEM AND TREATMENT

**S8**
RETURN HOME AFTER RECEIVING TREATMENT

**S13**
WRITE DOWN ALL RESULTS IN MEDICAL RECORD

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03012443 A2
- JP 2008139297 A
- JP 2009133813 A
- JP 2009287952 A
- JP 2008518617 PCT
- JP 2009156612 A

### Non-patent literature cited in the description

- *Admin: ''Rapid HIV Test by SD Bio-Line Kit,* 29 January 2008
- Revised Basic Photographic Engineering - Silver Halide Photography. Corona Publishing, Co., Ltd,
- **AKIRA SASAI.** Chemicals of Photography. Photography Industry Publishing Co., Ltd,
- **SHINICH KIKUCHI et al.** Handbook of Recent Prescription. AMIKO Publishing
- Staining Utilizing H2O2-POD System. *Clinical Examination,* vol. 41 (9), 1020-1024